# FASCICULE DE BREVET EUROPEEN

(11) **EP 2 869 827 B1**
(45) Date de publication et mention de la délivrance du brevet: **03.05.2017**
(21) Numéro de dépôt: 13737198.5
(22) Date de dépôt: 08.07.2013
(51) Int. Cl.: A61K 36/61, A61P 17/10, A61K 8/97, A61K 45/06, A61Q 19/00, A61K 31/122, A61K 31/19

(54) **UTILISATION D'UN EXTRAIT DE MYRTE EN TANT QU'AGENT ANTI-BIOFILM VIS-A-VIS DE P. ACNES**
VERWENDUNG EINES MYRTEEXTRAKTS ALS ANTI-BIOFILM-MITTEL GEGEN P. ACNES
USE OF A MYRTLE EXTRACT AS AN ANTI-BIOFILM AGENT AGAINST P. ACNES

(30) Priorité: 09.07.2012 FR 1256607
(43) Date de publication de la demande: 13.05.2015
(73) Titulaire: Pierre Fabre Dermo-Cosmétique, 92100 Boulogne-Billancourt (FR)
(72) Inventeur: LUC, Joelle, F-31150 Fenouillet (FR); FIORINI-PUYBARET, Christel, F-31500 Toulouse (FR)
(74) Mandataire: Regimbeau
(86) Numéro de dépôt international: PCT/EP2013/064415
(87) Numéro de publication internationale: WO 2014/009325

(56) Documents cités:
- WO-A2-2012/053010
- FR-A1- 2 783 425
- FIORINI-PUYBARET CHRISTEL ET AL: "Pharmacological Properties of Myrtacine (R) and Its Potential Value in Acne Treatment", PLANTA MEDICA, vol. 77, no. 14, septembre 2011 (2011-09), pages 1582-1589, XP002687346,

## Description

La présente invention concerne l'utilisation dans une composition dermatologique d'un extrait de myrte en tant qu'agent antibiofilm vis-à-vis de *Propionibacterium acnes.*

La présente invention concerne une composition dermatologique contenant un extrait de myrte pour son utilisation en tant qu'agent anti-adhésion des bactéries planctoniques de *Propionibacterium acnes* et/ou en tant qu'agent d'éradication ou de destruction des bactéries sessiles de *Propionibacterium acnes* organisées en biofilm, cette utilisation étant topique et destinée à diminuer la résistance à un antibiotique agissant sur *P*. *acnes*.

Cet extrait de myrte en tant qu'agent antibiofilm, s'est également révélé efficace pour diminuer la résistance aux antibiotiques classiquement administrés lors de la prise en charge de l'acné ou de la folliculite décalvante.

L'acné est une pathologie cutanée commune, résultat d'une inflammation des follicules pilo-sébacés due en grande partie à la colonisation de *Propionibacterium acnes* dans l'infundibulum.

La folliculite décalvante est une pustulose inflammatoire chronique du cuir chevelu responsable d'une alopécie cicatricielle résiduelle. La folliculite décalvante est une maladie orpheline dont la physiopathologie est mal connue, mais est probablement liée à une réaction inflammatoire à prédominance neutrophilique vis-à-vis de bactéries, probablement des bactéries de *Propionibacterium acnes.*

La bactérie *P. acnes* métabolise les triglycérides cutanés en acides gras irritants via des lipases qui agressent la paroi du follicule et du derme environnant, et produit également divers enzymes et chimioattractants des cellules phagocytaires de l'immunité qui aggravent l'inflammation.

La lutte contre cette espèce bactérienne est donc prioritaire dans le traitement de l'acné ou de la folliculite décalvante. L'usage de divers antimicrobiens topiques sont aujourd'hui largement utilisés (péroxyde de benzoyle, clindamycine, érythromycine, triclosan).

Une antibiothérapie systémique plus ou moins prolongée peut y être associée selon la gravité de l'affection (tétracyclines, doxycycline, minocycline, érythromycine).

On a constaté ces dernières années des échecs fréquents à ces traitements, dus à une forte proportion de souches résistantes de *P. acnes* à un ou plusieurs antibiotiques.

Cette résistance accrue peut être la conséquence d'une organisation des populations bactériennes planctoniques (en suspension) en biofilm. Les biofilms sont des communautés cellulaires bactériennes intégrées dans une matrice extracellulaire excrétée par les microorganismes, composée de polymères de sucres (EPS pour Extracellular Polymeric Substances) appelée glycocalyx. Le biofilm est donc une coque polysaccharidique qui isole la bactérie du milieu environnant. Celle-ci adapte son métabolisme, en le ralentissant. Le biofilm permet ainsi à la bactérie :
- d'adhérer aux kératinocytes de l'épithélium du follicule pilo-sébacé, ce qui facilite ses activités de modulation sur la différenciation et la prolifération kératinocytaire,
- de se développer dans le follicule pilo-sébacé en colonie, et
- de devenir résistante aux antibiotiques utilisés dans l'acné, notamment les cyclines et les macrolides

Le mode de vie en biofilm des bactéries est l'une des deux modalités de vie des organismes unicellulaires. Ce serait le mode de vie naturel de la plupart des microorganismes, notamment en milieu hostile. Le second mode de vie est la flottaison libre des bactéries dans un milieu liquide. Les bactéries associées au mode de vie en flottaison libre sont appelées bactéries planctoniques. Les bactéries associées au biofilm sont dites bactéries sessiles.

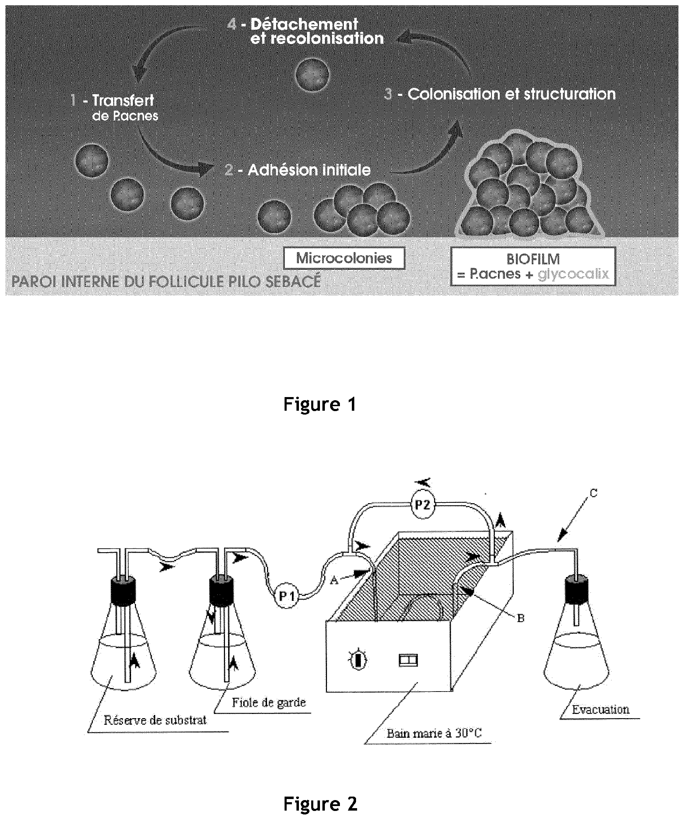

La formation, représentée par la Figure 1, du biofilm *P. acnes* dans le follicule pilo-sébacé se fait en quatre étapes :
1) transfert des *P. acnes* planctoniques vers la paroi interne du follicule Pilo-sébacé
2) adhésion initiale des *P. acnes sur la paroi*
3) colonisation et structuration des *P. acnes sessiles*
4) détachement des *P. acnes et recolonisation.*

Les bactéries sessiles (associées au biofilm) sont phénotypiquement et physiologiquement différentes des bactéries planctoniques (libres).

Cette organisation en biofilm a été évoquée pour expliquer les échecs thérapeutiques des traitements de l'acné (Burkart C.N., Burkart C.G., 2003. Microbiology's principle of biofilms as a major factor in the pathogenesis of acne vulgaris. Int. J. Dermatol.42. 925-927)

De récentes études ont confirmé l'aptitude de *P. acnes* à former des biofilms aussi bien :
➢ in vitro (Bayston R., Ashraf W., Barker-Davis R., Tucker E, Clement R., Clayton J., Freeman B.J., Nuradeen B. 2007. Biofilm formation by Propionibacterium acnes on biomaterials in vitro and in vivo: impact on diagnosis and treatment. J. Biomed. Mast. Res. A81, 705-709) (Coneye T., Peeters E., Nelis H.J. 2007a. Biofilm formation by Propionibacterium acnes is associated with increased resistance against antimicrobial agents and increased production of putative virulence factors. Res. Microbiol. 158. 386-392) (Holmberg A. Lood R, Mörgelin M. Söderquist B., Holst E. Collin M., Christensson B., Rasmussen M. 2009. Biofilm formation by Propionibacterium acnes is a characteristic of invasive isolates. Clin. Microbiol. Infect. 15, 787-795)
➢ qu'in vivo sur des dispositifs médicaux (Coneye T., Honraet K., Rossel B. Nelys HJ Biofilm in skin infection : Propionibacterium acnes et acne vulgaris. Infect Disord Drug Target 2008. 14. 649-659) (Ramage G, Tunney MM, Patrick S, Gorman SP and Nixon JR. 2003.Formation of Propionibacterium acnes biofilms on orthopaedic biomaterials and their susceptibility to antimicrobials. Biomaterials; 24: 3221-3227) (Craig G., Burkhart MD, Craig N. 2007. Expanding the microcomedone theory and acne therapeutics: Propionibacterium acnes biofilm produces biological glue that hold cornéocytes together to form plug. J Am Acad Dermatol. October).

Les technologies même récentes ne permettent pas de visualiser le biofilm in situ dans les follicules, mais une étude récente a permis de détecter la présence de macro-colonies/biofilm à *P. acnes* dans les follicules pilo-sébacés à partir de biopsies cutanées (Jahns AC, Lundskog B., Ganceviciene R., Palmer RH, Golovleva I, Zouboulis CC, McDowell A, Patrick S., Alexeyev OA. 2012. An increased incidence of Propionibacterium acnes biofilms in acne vulgaris : a case-control study. Br J Dermatol. Feb 22).

Le concept du biofilm à *P. acnes,* aujourd'hui majoritairement admis ouvre la voie à de nouvelles cibles pour la thérapie, telles que : l'inhibition de la formation du biofilm, l'inhibition de la dissémination ou encore l'éradication chimique ou mécanique du biofilm installé.

Ces voies représentent une alternative à l'activité biocide classiquement utilisée pour combattre l'installation des microorganismes. Ces 2 types d'activité, biocide et anti-biofilm mettent en jeu des propriétés différentes, l'une ne préfigurant pas l'autre.

La présente invention vise à démontrer l'activité de l'extrait de Myrte, d'une part sur l'inhibition de la formation du biofilm à *P. acnes,* et d'autre part sur l'inhibition de la dissémination ou l'éradication (ou destruction) du biofilm établi.

La présente invention concerne les travaux menés sur l'espèce *Myrtus communis.*

Préférentiellement, l'extrait est réalisé à partir des feuilles de *Myrtus communis.*

De préférence, il s'agit d'une fraction apolaire de parties aériennes de myrte.

L'extrait selon la présente invention peut être obtenu par extraction à l'aide d'un solvant ou mélange de solvants choisi parmi
- les alcools tels que l'éthanol, le méthanol, l'isopropanol
- les cétones dont l'acétone et la méthyléthylacétone
- l'hexane
- le chlorure de méthylène
- l'éther isopropylique
- l'acétate d'éthyle ;
ou par CO2 supercritique.

L'extrait peut également être stabilisé par addition d'un antioxydant comme par exemple le butylhydroxytoluène ou de l'alpha tocophérol à des quantités comprises entre 0,05 à 1 g%g d'extrait sec.

L'extrait selon la présente divulgation présente la caractéristique d'être riche en myrtucommulones et en en acide ursolique. Les myrtucommulones A, B', D, B, isoS (isosemimyrtucommulone) et S (semimyrtucommulone) sont les principales myrtucommulones présentes.

Avantageusement, la teneur en myrtucommulones totales dans l'extrait est comprise entre 3 et 10% en poids.

La teneur en acide ursolique est comprise entre 10 et 30%, de préférence ≥ 15 % en poids.

Ces molécules portent l'activité revendiquée dans le cadre de la présente invention.

Dans un mode de réalisation particulier de l'invention, l'extrait de myrte sera de préférence tel que décrit dans le brevet EP 1 112 079. Ce document décrit les propriétés antibactériennes de l'extrait de Myrtus communis et ses applications dans des compositions cosmétiques ou dermatologiques.

Un point tout à fait remarquable de la présente invention est que l'activité antibiofilm de la composition selon l'invention est observée même à très faibles concentrations en extrait de myrte ; soit à partir de 0,01% en poids par rapport au poids total de la composition.

La quantité d'extrait dans la composition est comprise entre 0,01 % et 1% et de préférence 0,03 % et 1%.

Avantageusement, la quantité d'extrait est inférieure ou égale à 0,1%.

Plus particulièrement, la quantité d'extrait est supérieure ou égale à 0,01 % et inférieure ou égale à 0,1%. De façon encore plus préférentielle, la quantité d'extrait est supérieure ou égale à 0,03 % et inférieure ou égale à 0,1%.

Par « agent antibiofilm vis-à-vis de *Propionibacterium acnes* », on entend au sens de la présente invention un agent permettant :
➢ d'inhiber l'adhésion du biofilm de *Propionibacterium acnes* (éviter la formation du biofilm de *Propionibacterium acnes*),et/ou
➢ d'éradiquer le biofilm de *Propionibacterium acnes* établi (destruction, détachement).

Afin de démontrer l'activité d'agent antibiofilm, un modèle dynamique de formation du biofilm sur support inerte, déjà utilisé dans d'autres axes (Campanac C., Pineau L., Payard M., Baziard-Mouysset G., Roques C. 2002. Interaction between biocide cationic agents and bacterial biofilms. Antimicrob. Ag. Chemother. May 46 (5) 1469-1474), a été spécifiquement décliné sur *P. acnes.* Ainsi, les deux aspects évoqués ont été explorés :
➢ Influence de l'extrait sur la génèse du biofilm à *P. acnes* : cette activité met en jeu les structures impliquées dans l'adhésion des cellules sur une surface ainsi que dans la structuration du biofilm,
➢ Sur l'éradication du biofilm déjà établi mettant en jeu d'autres propriétés (détersion...).

L'extrait de myrte selon la divulgation contribue à lutter contre *P. acnes* en limitant son organisation en biofilm. Cette action anti-adhésion des bactéries planctoniques de *P. acnes* et destructurante du biofilm établi de *P. acnes* va favoriser la disparition des lésions acnéiques des peaux acnéiques et/ ou des peaux grasses à tendance acnéique.

La Demanderesse a poursuivi ses recherches sur l'utilisation de l'extrait de myrte comme agent anti biofilm, et a relevé de façon surprenante et inattendue que l'extrait de myrte permet de diminuer la résistance de souches de *P. acnes* - préférentiellement les souches de *P. acnes* organisées en biofilm - aux antibiotiques administrés agissant sur *P. acnes,* préférentiellement sous forme topique.

La présente invention décrit donc une composition dermatologique contenant un extrait de myrte à une concentration supérieure ou égale à 0,01 % en poids, pour son utilisation en tant qu'agent antibiofilm vis-à-vis de *Propionibacterium acnes.*

Avantageusement, la présente invention décrit une composition dermatologique contenant un extrait de myrte à une concentration supérieure ou égale à 0,01 % en poids, pour son utilisation en tant qu'agent anti-adhésion des bactéries planctoniques de *Propionibacterium acnes* et/ou en tant qu'agent d'éradication ou de destruction des bactéries sessiles de *Propionibacterium acnes* organisées en biofilm.

En particulier, la présente invention décrit une composition dermatologique contenant un extrait de myrte à une concentration supérieure ou égale à 0,01 % en poids, pour son utilisation en tant qu'agent anti-adhésion des bactéries planctoniques pour favoriser leur flottaison libre.

Plus particulièrement, la présente invention décrit une composition dermatologique contenant un extrait de myrte à une concentration supérieure ou égale à 0,01 % en poids, pour son utilisation anti-biofilm préventive contre l'adhésion des bactéries planctoniques.

Plus particulièrement, la présente invention décrit une composition dermatologique contenant un extrait de myrte à une concentration supérieure ou égale à 0,01 % en poids, pour son utilisation préventive contre la réorganisation des bactéries planctoniques en biofilm après sa destruction.

Un autre aspect de la présente invention décrit une composition contenant un extrait de myrte à une concentration de 0,01 % à 1% en poids.

Selon un autre aspect de la présente invention, la quantité d'extrait est supérieure ou égale à 0,01 % et inférieure ou égale à 0,1 % en poids.

Selon un autre aspect de la présente invention, l'extrait de myrte est une fraction apolaire comprenant les myrtucommulones et de l'acide ursolique.

L'utilisation topique de la composition selon la présente invention est destinée à diminuer la résistance à un antibiotique préférentiellement administré sous forme topique et agissant sur *P. acnes.*

Selon un autre aspect de la présente invention, la composition est utilisée dans le traitement de l'acné ou de la folliculite décalvante.

Selon un autre aspect de la présente invention, elle concerne un produit de combinaison d'une composition dermatologique contenant un extrait de myrte à une concentration de 0,01 % à 1% en poids et d'une composition dermatologique contenant au moins un antibiotique, en particulier, un antibiotique topique actif sur *P. acnes* destiné à une utilisation simultanée, séparée ou étalée dans le temps pour le traitement de l'acné ou de la folliculite décalvante.

La composition dermatologique du produit de combinaison selon l'invention, contient une quantité d'extrait de myrte supérieure ou égale à 0,01 % et inférieure ou égale à 0,1% en poids.

Selon un autre aspect de l'invention, l'extrait de myrte est à une concentration de 0,1 % en poids.

Lesdits antibiotiques classiquement utilisés sont l'érythromycine, la doxycycline et la clindamycine.

L'agent antibiofilm selon l'invention améliore l'efficacité thérapeutique de l'antibiotique.

Les antibiotiques mentionnés ci-dessus associés à l'extrait de myrte, présentent des concentrations minimales inhibitrices (CMI) inférieures à celles des antibiotiques seuls.

La CMI est la plus faible concentration de produit inhibant la croissance des bactéries.

Dans le cadre du traitement de l'acné, il y a un réel intérêt d'associer une antibiothérapie avec l'application d'une composition à base d'un extrait de myrte en tant qu'agent antibiofilm. Cela permet en effet de diminuer la résistance de certaines souches de *P. acnes* à l'érythromycine ou clindamycine.

### FORMULATION / GALENIQUE

La composition selon la présente divulgation peut se présenter sous toute forme adaptée à l'application topique : crème, gel, stick, sérum.

L'invention pourra être mieux comprise à l'aide des exemples et évaluations pharmacologiques suivants :

### EXEMPLES

### 1- préparation d'extrait

### 1.1- Exemple 1 :

1 kg de feuilles de Myrte broyées sont extraites par 10 volumes d'acétate d'isopropyle sous agitation à reflux. Extraire à reflux pendant 1 heure sous agitation. Après filtration et rinçage du marc, les jus d'extraction sont décolorés par addition de charbon actif. Après filtration, le filtrat décoloré est concentré jusqu'à 2 litres puis séché sur eau jusqu'à l'élimination de l'acétate d'isopropyle. La phase aqueuse obtenue est ensuite séchée par lyophilisation.

1 kg de feuille de myrte permet d'obtenir 25 g environ d'extrait sec de myrte. Celui-ci renferme 7 % de myrtucommulones et 25 % d'acide ursolique.

### 1.2- Exemple 2

1 kg de feuilles de Myrte broyées sont extraites par 5 volumes d'acétate d'isopropyle sous agitation à reflux pendant 1 heure. Après filtration et rinçage du marc, les jus d'extraction sont décolorés par addition de charbon actif. Après filtration, le filtrat décoloré est concentré jusqu'à 2 litres puis séché sur éthanol jusqu'à l'élimination de l'acétate d'isopropyle. La phase aqueuse obtenue est ensuite désodorisée par traitement thermique, puis séchée par lyophilisation.

1 kg de feuille de myrte permet d'obtenir 25 g environ d'extrait sec de myrte. Celui-ci renferme 7 % de myrtucommulones et 25 % d'acide ursolique.

### 2- formulations

### 2.1- Exemple 3 : FORMULE gel nettoyant

Zinc coceth sulfate (Zetesol Zn®, Zchimmer&Schwarz) de 5 à 20%
DECYL GLUCOSIDE
BETAINE LAURYL
Polysorbate 20 de 0.5 à 4%
PEG-5 ETHYLHEXANOATE de 1 à 3%
Cétéareth-60 myristyl glycol de 0.5 à 3%
SALICYLATE ZINC de 0.1 à 0.5%
EXT.SERENOA REPENS de 0.1 à 0.3 %
EXT. SEC LIPOPHILE MYRTE de 0.01 à 0.1%
BENZOATE SODIUM / CITRIQUE ACIDE / HYDROXYDE SODIUM qsp

### 2.2- Exemple 4 : Crème

Extrait de myrte 0,03 à 0,1%
AHA BHA 1 à 8%
Xanthane gum 0,1%
Diméthicone 1%
Cétostéaryl Isononanoate 12%
Glycéryl stéarate 2%
Cetearyl Alcool & Ceteareth-33 6%
Polymethylméthacrylate 2%
Eau qsp 100%

### EVALUATION

### - Obtention d'un biofilm à P. acnes en flux continu

Le montage permettant d'obtenir la formation du biofilm est décrit sur la figure 2.

Les deux pompes utilisées servent respectivement:
- à l'alimentation de la boucle en substrat (pompe N°1),
- à l'agitation du contenu du circuit (pompe N°2).

L'ensemble du montage expérimental décrit dans la figure 2 est placé sous une hotte à flux laminaire.

Le circuit est alimenté avec un milieu de culture. L'inoculation est ensuite réalisée en injectant au niveau du point B, une quantité de suspension bactérienne de *P. acnes CIP* 53117 pour obtenir une concentration finale en microorganismes d'environ 10⁷ UFC/mL.

On répartit les microorganismes de l'inoculum dans la totalité de la boucle = phase d'homogénéisation suivie d'une alimentation en substrat par la pompe N°1.

Après 48 heures de formation, un biofilm à P. *acnes CIP 53117* est obtenu.

Dans le dispositif expérimental illustré à la figure 2, les références au dessin ont les significations suivantes :
P1: Pompe d'alimentation en substrat
P2: Pompe servant à l'agitation du circuit
A-B: Zone de prélèvement des portions de tube
B: Zone d'inoculation de la boucle.
A-B+ Portion de tube contenant la pompe P2.

### EVALUATION de l'inhibition d'adhésion de l'extrait de myrte sur la formation d'un biofilm à P. acnes.

Afin d'évaluer l'effet inhibiteur d'adhésion de l'extrait sec de myrte préparé selon l'exemple 2 sur la formation d'un biofilm à *P. acnes CIP* 53117, deux montages ont été préparés comme décrit dans le paragraphe précédent. Le premier servira de témoin. Le second sera alimenté en milieu de culture additionné d'extrait sec de myrte à la concentration finale de 0,1% (concentration non létale). L'essai est réalisé deux fois.

Des dénombrements des bactéries adhérées et planctoniques sont réalisés aux temps 5h, 24h et 48 h, sur le support « traité » versus un témoin sans traitement selon le protocole suivant. L'échantillon de tube est découpé longitudinalement en quatre tronçons identiques et les surfaces internes de chacune de ces portions sont raclées à l'aide d'une lame de scalpel stérile dans 10ml de diluant.

Les portions du tube et le liquide sont ensuite transférés dans un tube à essai et soumis à une agitation pendant environ 1 minute.

Puis un dénombrement - par étalement - des bactéries est réalisé sur une gélose de culture.

Aux mêmes temps un échantillon d'effluent est récupéré pour dénombrement des bactéries planctoniques, selon le même protocole.

Les boites sont placées en incubation à 37°C ± 2°C, sous anaérobiose.

Les UFC sont dénombrées après 96 heures d'incubation.

Les résultats sont exprimés en UFC/cm² pour les bactéries adhérées et en UFC/ml d'effluent pour les bactéries planctoniques.

### RESULTATS

Les résultats obtenus sont présentés dans le tableau 1.

L'estimation de la population cultivable adhérée est exprimée en log UFC/cm² de tube et celle de la population cultivable planctonique en log UFC/mL.

**Tableau 1. Résultats de l'inhibition d'adhésion de P. acnes par l'extrait sec de myrte à 0,1% (P/V). Résultats exprimés en Log (UFC/cm² ou mL)**

| Temps de Formation | Essai | | Témoin | Extrait sec de myrte 0,1% (P/V) | |
|---|---|---|---|---|---|
| | | Adhérées | Planctoniques | Adhérées | Planctoniques |
| | | Log | Log (UFC/mL) | Log | Log (UFC/mL) |
| | | (UFC/cm²) | | (UFC/cm²) | |
| | Essai 1 | 4,28 | 5,11 | 3,23 | 4,04 |
| 5 heures | Essai 2 | 4,57 | 4,97 | 3,20 | 4,51 |
| | Moyenne | 4,42 | 5,04 | 3,22 | 4,27 |
| | Essai 1 | 4,08 | 3,67 | 0,70 | 3,93 |
| 24 heures | Essai 2 | 4,77 | 3,18 | 2,77 | 3,32 |
| | Moyenne | 4,43 | 3,42 | 1,73 | 3,63 |
| | Essai 1 | 4,18 | 2,38 | 2,04 | 1 |
| 48 heures | Essai 2 | 3,46 | 3,23 | 0,48 | 1,08 |
| | Moyenne | 3,82 | 2,81 | 1,26 | 1,04 |

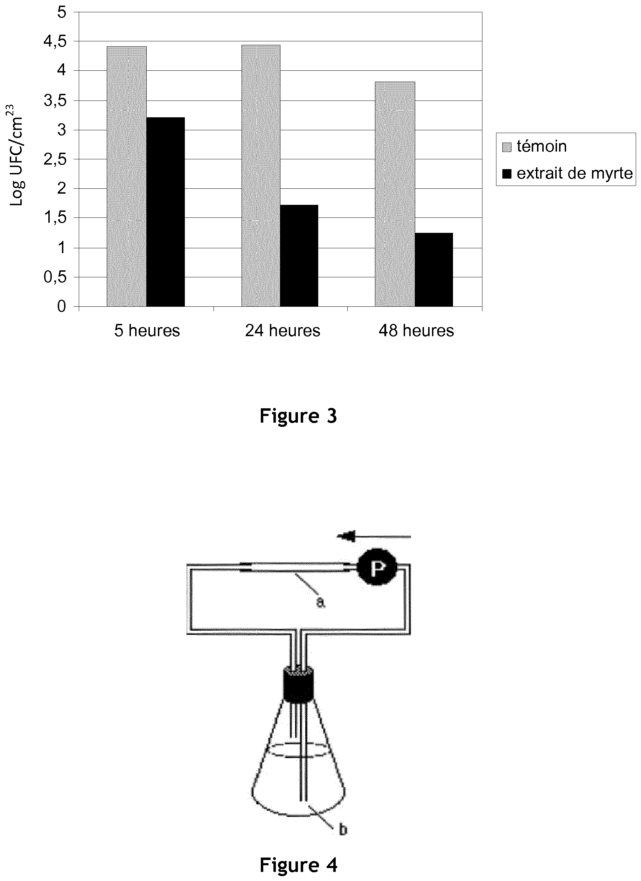

Les moyennes des résultats obtenus sur les populations adhérées, exprimées en log UFC/cm², sont représentées dans la figure 3 ci-dessous.

Une inhibition de l'adhésion de *P. acnes est* observée 5 heures après l'inoculation pour le montage avec de l'extrait sec de myrte. Cette inhibition est de l'ordre de 1,2 log. L'estimation de la population planctonique est du même ordre entre les deux montages (traité et témoin). Après 24 heures de formation, le phénomène se maintient avec une inhibition de l'ordre de 2,7 log. Après 48 heures, cette inhibition est restée constante, de l'ordre de 2,6 log. Pendant ce laps de temps, une baisse importante de la population planctonique cultivable est observée pour les deux montages (traité et témoin).

Ces résultats démontrent une inhibition d'adhésion dans les conditions testées induite par l'extrait sec de myrte à 0,1% (P/V).

### EVALUATION DE l'activité détachante

Afin d'évaluer l'activité détachante de l'extrait sec de myrte préparé selon l'exemple 2 sur un biofilm à P. *acnes CIP* 53117 obtenu après 48 heures de formation comme décrit précédemment.

Les conditions d'essais (cf. figure 4) sont les suivantes :
- Activité détachante de l'extrait sec de myrte à 0,1% (P/V) versus l'eau distillée stérile,
- Temps de contact : 1, 5 et 10 minutes de contact,
- Température ambiante.

Après chaque temps de contact définis, différents prélèvements sont réalisés :
- un échantillon de tube pour dénombrement des bactéries résiduelles (2 cm),
- un échantillon de la solution de circulation pour dénombrement des bactéries remises en suspension,

Dans le dispositif expérimental illustré à la figure 4, les références ont les significations suivantes :
a = échantillon de tube pour dénombrement des bactéries résiduelles
b = solution de circulation
P = pompe.

L'essai est réalisé en duplicate pour l'extrait sec de myrte et une fois pour l'eau distillée stérile.

Un dénombrement du nombre de microorganismes adhérés à été réalisé avant de débuter les différents traitements (témoin).

### RESULTATS

Les résultats obtenus sont présentés dans le tableau 2.
L'estimation de la population cultivable adhérée est exprimée en log UFC/cm² de tube et celle de la population cultivable planctonique en log UFC/200 mL (volume de la solution de circulation).

**Tableau 2: Résultats de l'activité détachante d'une solution d'extrait sec de myrte à 0,1% (P/V) versus l'eau distillée stérile (EDS) sur un biofilm à P. acnes (après 48h de formation). Résultats exprimés en Log (UFC/cm² ou mL).**

| Temps de contact | Essai | Eau distillée stérile Adhérées Log (UFC/cm²) | Extrait sec de myrte 0,1% (P/V) Adhérées Log (UFC/cm²) |
|---|---|---|---|
| | Essai 1 | 4,46 | 4,46 |
| To | Essai 2 | 4,23 | 4,23 |
| Moyenne | Moyenne | 4,35 | 4,35 |
| | Essai 1 | 3,28 | 1,00 |
| 1 minute | Essai 2 | - | 1,72 |
| | | 3,28 | 1,36 |
| | Essai 1 | 4,00 | 0,90 |
| 5 minutes | Essai 2 | - | 0,48 |
| Moyenne | Moyenne | 4,00 | 0,69 |
| | Essai 1 | 3,30 | 0,48 |
| 10 minutes | Essai 2 | | 0,90 |
| Moyenne | Moyenne | 3,30 | 0,69 |

La série témoin réalisée avec de l'eau distillée stérile a permis de valider l'essai puisque aucune réduction notable de la population cultivable adhérée n'est observée (< 1 log de réduction = élimination des premières couches superficielles du biofilm).

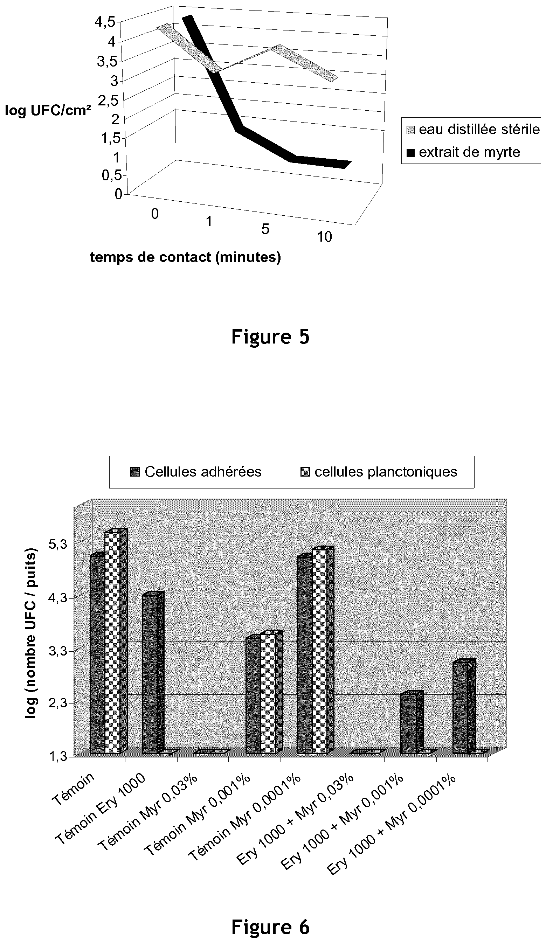

L'activité détachante d'une solution d'extrait sec de myrte à 0,1% (P/V) versus l'eau distillée stérile sur un biofilm à *P. acnes* (après 48h de formation) exprimés en log (UFC/cm²) est illustrée à la figure 5.

En présence d'extrait sec de myrte à 0,1% (P/V), une réduction significative de la population adhérée est observée. Elle est de l'ordre de 3 log après 1 minute de contact.
L'allongement du temps de circulation de la solution de 1 à 5 minutes permet un gain supplémentaire en terme de détachement de l'ordre de 0,7 log.

### EVALUATION de l'activité de l'association érythromycine/extrait de myrte sur un biofilm à P. acnes

L'extrait de myrte est préparé selon l'exemple 2.

### IDENTIFICATION DES SOUCHES

Quatre souches de *Propionibacterium* acnes ont été retenues :
2 SOUCHES résistantes à l'érythromycine (ERY R)
   - *Propionibacterium* acnes CIP110371
   - isolat clinique R4
2 souches sensibles à l' érythromycine (ERY S)
   - *Propionibacterium* acnes CIP53117T
   - isolat clinique B872

### PROTOCOLE

Une suspension titrée à environ 10⁶ UFC/ml de *P acnes,* dans un bouillon minimal ne permettant aucune croissance bactérienne, a été déposée dans des cupules d'une microplaque à 24 puits.

Les microplaques sont placées en conditions anaérobies à 37 ± 2°C pendant 48h, le temps de formation d'un biofilm établi.

Après 48h, le surnageant de chaque puits est éliminé, puis les cupules rincées. L'érythromycine et l'extrait de myrte dans différentes concentrations sont ajoutés pour un contact de 24h sur les cellules adhérées (soit 72 heures d'adhésion au total). La concentration en érythromycine choisie est de 1000µg/ml. A cette concentration, il a été vérifié, sur les bactéries isolées, que cet antibiotique avait une activité bactéricide (entre 4 et 5 log de réduction en 24 heures) vis-à-vis des 2 souches ery sensibles et n'avait pas d'activité bactéricide vis-à-vis des 2 souches ERY R.

Après 72 h d'adhésion
- dénombrement des cellules planctoniques
- dénombrement des cellules adhérées (sessiles) après rinçage du puits puis raclage dans le tryptone-sel
   Des témoins ont été réalisés en parallèle (cellules adhérées + planctoniques)
- témoin biofilm 72H (avec étape de renouvellement du milieu à 48h)
- témoin myrte aux différentes concentrations (ajout après 48h/ contact 24h)
- témoin érythromycine 1000µg/ml (ajout à 48h / contact 24h)
- témoin extrait myrte aux concentrations testées

Pour tous les puits en contact avec l'érythromycine, 1mL de chaque dilution est filtré sur membrane (Microfil®, Millipore) puis le filtre est rincé 3 fois à l'aide de 50mL d'eau distillée stérile. Les membranes sont déposées sur gélose Columbia + 5% de sang de mouton. Les boites sont placées en incubation à 37°C ± 2°C, sous anaérobiose. Après 72h à 96h d'incubation, les UFC (Unité Formant Colonie) sont dénombrées.
Pour tous les témoins biofilm et extrait de myrte seul, 100µL de chaque dilution sont ensemencés par étalement sur gélose Columbia + 5% de sang de mouton. Les boites sont placées en incubation à 37°C ± 2°C, sous anaérobiose. Après 72h à 96h d'incubation, les UFC sont dénombrées.

### RESULTATS

Cet essai a consisté à évaluer l'activité de l'érythromycine (1000 µg/mL) combiné à l'extrait de myrte, aux concentrations de 0,03%, 0,001% et 0,0001%. Les résultats sont exprimés en log de la population dénombrée.

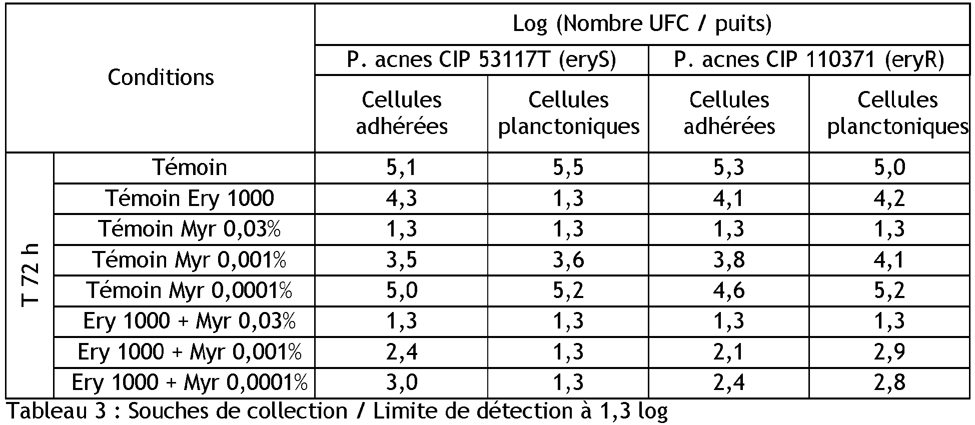

### Résultats ERY S - souches de collection

L'activité de l'érythromycine (1000 µg/mL) combiné à l'extrait de myrte, aux concentrations de 0,03%, 0,001% et 0,0001% est illustrée à la Figure 6.

### Résultats ERY R - souches de collection

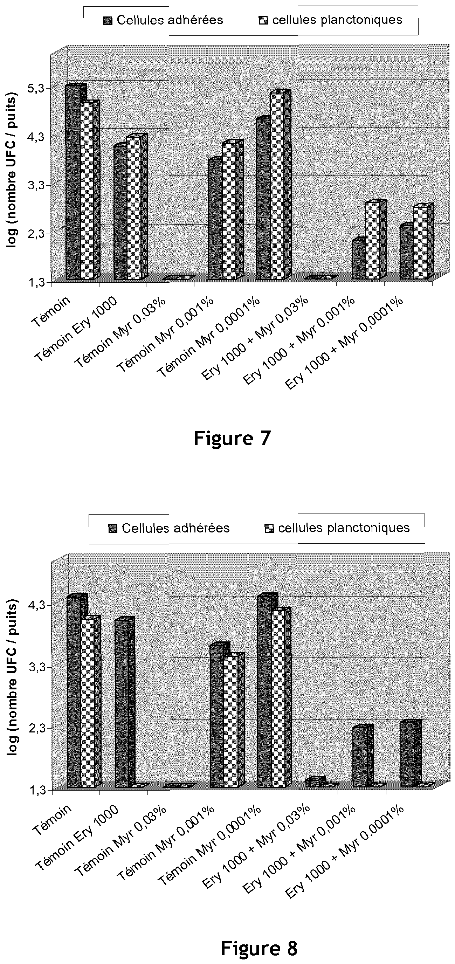

L'activité de l'érythromycine (1000 µg/mL) combiné à l'extrait de myrte, aux concentrations de 0,03%, 0,001% et 0,0001% est illustrée à la Figure 7.

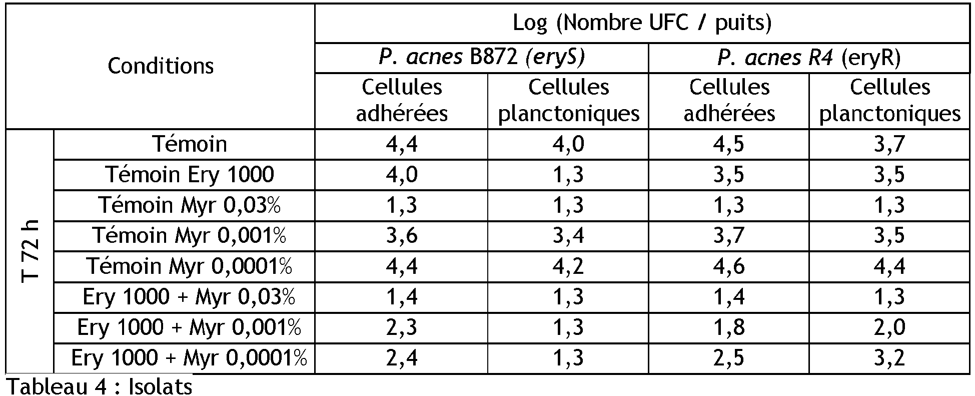

### Résultats ERY S - Isolats

L'activité de l'érythromycine (1000 µg/mL) combiné à l'extrait de myrte, aux concentrations de 0,03%, 0,001% et 0,0001% est illustrée à la Figure 8.

### Résultats ERY R - Isolats

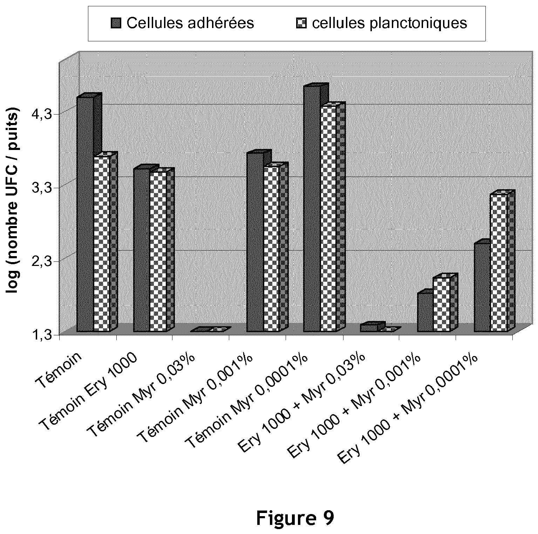

L'activité de l'érythromycine (1000 µg/mL) combiné à l'extrait de myrte, aux concentrations de 0,03%, 0,001% et 0,0001% est illustrée à la Figure 9.

### CONCLUSION GENERALE :

### - Extrait seul :

Une activité bactéricide importante de l'extrait de myrte seul est observée pour la concentration de 0,03% aussi bien vis à vis des souches eryS que des souches eryR.

Les résultats obtenus illustrent également que l'extrait de myrte seul présente une activité significative - certes sur les bactéries planctoniques - mais aussi sur les bactéries *P acnes* adhérées (effet dose dépendant).

Les concentrations moins actives en extrait de myrte (0,001% et 0,001%) ont été choisies pour pouvoir mettre en évidence les effets de la combinaison extrait de myrte - traitement antibiotique type érythromycine.

### - Erythromycine seule :

Les résultats confirment que l'organisation des bactéries *P acnes* en biofilm (eryS et eryR) diminue leur sensibilité à l'érythromycine.

Il a en outre été vérifié que les souches eryR planctoniques présentent une résistance à l'érythromycine.

Enfin, la bactéricidie de l'érythromycine est importante sur les souches eryS.

### - Association extrait de myrte et érythromycine :

Une destruction du biofilm (diminution du nombre de bactéries de *P acnes* organisées en biofilm) et une élimination d'une partie de la population planctonique (bactéries détachées du biofilm) sont observées pour les 3 couples évalués. Un gain d'au moins 1 log de réduction de la population adhérée est observé pour les concentrations les plus faibles en extrait de myrte (0,001% et 0,0001%) associé à l'érythromycine, en comparaison de l'érythromycine seule ou de l'extrait seul. Pour la concentration en extrait de myrte supérieure (0,03%), l'effet d'une synergie est masqué par la forte activité de l'extrait.

Ainsi, une potentialisation d'action a bien été observée en combinant un extrait de myrte à un antibiotique de référence. En effet, l'extrait de myrte permet de diminuer la résistance des souches *P acnes* biofilmées à la molécule de référence en antibiothérapie. L'extrait de myrte optimise l'action de l'érythromycine vis-à-vis des bactéries *P acnes.*

## Revendications

1. Composition dermatologique contenant un extrait de myrte à une concentration de 0,01% à 1% en poids, pour son utilisation en tant qu'agent anti-adhésion des bactéries planctoniques de *Propionibacterium acnes* et/ou en tant qu'agent d'éradication ou de destruction des bactéries sessiles de *Propionibacterium acnes* organisées en biofilm,
cette utilisation étant topique et destinée à diminuer la résistance à un antibiotique agissant sur *P. acnes.*

2. Composition pour son utilisation selon la revendication 1, **caractérisée en ce que** l'utilisation est une utilisation en tant qu'agent anti-adhésion des bactéries planctoniques pour favoriser leur flottaison libre.

3. Composition pour son utilisation selon la revendication 2, **caractérisée en ce que** l'utilisation est une utilisation anti-biofilm préventive contre l'adhésion des bactéries planctoniques.

4. Composition pour son utilisation selon la revendication 2, **caractérisée en ce que** l'utilisation est une utilisation préventive contre la réorganisation des bactéries planctoniques en biofilm après sa destruction.

5. Composition pour son utilisation selon les revendications 1 à 4, **caractérisée en ce que** la quantité d'extrait est supérieure ou égale à 0,01 % et inférieure ou égale à 0,1% en poids.

6. Composition pour son utilisation selon l'une des revendications 1 à 5, **caractérisée en ce que** l'extrait de myrte est une fraction apolaire comprenant les myrtucommulones et de l'acide ursolique.

7. Composition pour son utilisation selon la revendication 6, **caractérisée en ce que** lesdites myrtucommulones comprennent principalement les myrtucommulones A, B', D, B, l'isosemimyrtucommulone et la semimyrtucommulone, avantageusement à une teneur en myrtucommulones totales comprise entre 3% et 10% en poids, par rapport au poids total de l'extrait de myrte.

8. Composition pour son utilisation selon la revendication 6, **caractérisée en ce que** la teneur en acide ursolique est comprise entre 10% et 30% en poids, par rapport au poids total de l'extrait de myrte.

9. Composition pour son utilisation selon la revendication 6, **caractérisée en ce que** la teneur en acide ursolique est inférieure à 15% en poids, par rapport au poids total de l'extrait de myrte.

10. Composition pour son utilisation selon l'une des revendications 1 à 9, **caractérisée en ce que** l'antibiotique est administré sous forme topique.

11. Composition pour son utilisation selon l'une des revendications 1 à 10, **caractérisée en ce que** la composition est utilisée dans le traitement de l'acné.

12. Composition pour son utilisation selon l'une des revendications 1 à 11, **caractérisée en ce que** la composition est utilisée dans le traitement de la folliculite décalvante.

13. Produit de combinaison d'une composition dermatologique contenant un extrait de myrte à une concentration de 0,01% à 1% en poids et d'une composition dermatologique contenant au moins un antibiotique, destiné à une utilisation simultanée, séparée ou étalée dans le temps pour le traitement de l'acné, ou de la folliculite décalvante.

14. Produit selon la revendication 13, **caractérisée en ce que** la composition dermatologique contient une quantité d'extrait de myrte supérieure ou égale à 0,01 % et inférieure ou égale à 0,1% en poids.

## Patentansprüche

1. Dermatologische Zusammensetzung, enthaltend einen Myrtheextrakt in einer Konzentration von 0,01 Gew.-% bis 1 Gew.-%, für deren Verwendung als Anti-Adhäsions-Mittel von Planktonbakterien von *Propionibacterium acnes* und/oder als Beseitigungs- oder Zerstörungsmittel von sessilen, als Biofilm organisierten Bakterien von *Propionibacterium acnes,*
wobei diese Verwendung topisch ist und bestimmt, um die Resistenz auf ein auf *P. acnes* wirkendes Antibiotikum zu verringern.

2. Zusammensetzung für deren Verwendung nach Anspruch 1, **dadurch gekennzeichnet, dass** die Verwendung eine Verwendung als Anti-Adhäsions-Mittel von Planktonbakterien ist, um deren freies Floaten zu fördern.

3. Zusammensetzung für deren Verwendung nach Anspruch 2, **dadurch gekennzeichnet, dass** die Verwendung eine vorbeugende Anti-Biofilm-Verwendung gegen die Adhäsion von Planktonbakterien ist.

4. Zusammensetzung für deren Verwendung nach Anspruch 2 , **dadurch gekennzeichnet, dass** die Verwendung eine vorbeugende Verwendung gegen die Reorganisierung der Planktonbakterien als Biofilm nach seiner Zerstörung ist.

5. Zusammensetzung für deren Verwendung nach den Ansprüchen 1 bis 4, **dadurch gekennzeichnet, dass** die Extraktmenge über oder gleich 0,01 Gew.-% und unter oder gleich 0,1 Gew.-% ist.

6. Zusammensetzung für deren Verwendung nach einem der Ansprüche 1 bis 5, **dadurch gekennzeichnet, dass** der Myrtheextrakt eine apolare Fraktion ist, umfassend die Myrtucommulone und Ursolsäure.

7. Zusammensetzung für deren Verwendung nach Anspruch 6, **dadurch gekennzeichnet, dass** die Myrtucommulone hauptsächlich die Myrtucommulone A, B', D, B, das Isosemimyrtucommulon und das Semimyrtucommulon, in vorteilhafter Weise in einem Gesamt-Myrtucommulonegehalt zwischen 3 Gew.-% und 10 Gew.-% inklusive im Verhältnis zum Gesamtgewicht des Myrtheextrakts, enthalten.

8. Zusammensetzung für deren Verwendung nach Anspruch 6, **dadurch gekennzeichnet, dass** der Ursolsäuregehalt zwischen 10 Gew.-% und 30 Gew.-% inklusive im Verhältnis zum Gesamtgewicht des Myrtheextrakts beträgt.

9. Zusammensetzung für deren Verwendung nach Anspruch 6, **dadurch gekennzeichnet, dass** der Ursolsäuregehalt unter 15 Gew.-% im Verhältnis zum Gesamtgewicht des Myrtheextrakts beträgt.

10. Zusammensetzung für deren Verwendung nach einem der Ansprüche 1 bis 9, **dadurch gekennzeichnet, dass** das Antibiotikum in topischer Form verabreicht wird.

11. Zusammensetzung für deren Verwendung nach einem der Ansprüche 1 bis 10, **dadurch gekennzeichnet, dass** die Zusammensetzung bei der Aknebehandlung verwendet wird.

12. Zusammensetzung für deren Verwendung nach einem der Ansprüche 1 bis 11, **dadurch gekennzeichnet, dass** die Zusammensetzung bei der Behandlung von Folliculitis decalvans verwendet wird.

13. Kombinationsprodukt einer dermatologischen Zusammensetzung, enthaltend einen Myrtheextrakt in einer Konzentration von 0,01 Gew.-% bis 1 Gew.-%, und einer dermatologischen Zusammensetzung, enthaltend mindestens ein Antibiotikum, das für eine gleichzeitige, getrennte oder zeitlich versetzte Verwendung für die Behandlung der Akne oder von Folliculitis decalvans bestimmt ist.

14. Produkt nach Anspruch 13, **dadurch gekennzeichnet, dass** die dermatologische Zusammensetzung einen Myrtheextrakt über oder gleich 0,01 Gew.-% und unter oder gleich 0,1 Gew.-% enthält.

## Claims

1. A dermatological composition containing myrtle extract at a concentration of 0.01 % to 1 % by weight, for use as an anti-adhesion agent against planktonic *Propionibacterium acnes* bacteria and/or as an agent for eradicating or destroying sessile *Propionibacterium acnes* bacteria organized into biofilm,
this use being topical and intended to decrease resistance to an antibiotic acting on *P. acnes.*

2. The composition for use according to claim 1, **characterized in that** the use is use as an anti-adhesion agent against planktonic bacteria in order to promote the free-floating thereof.

3. The composition for use according to claim 2, **characterized in that** the use is a preventive antibiofilm use against the adhesion of planktonic bacteria.

4. The composition for use according to claim 2, **characterized in that** the use is a preventive use against the reorganization of planktonic bacteria into biofilm once a biofilm is destroyed.

5. The composition for use according to claims 1 to 4, **characterized in that** the quantity of extract is greater than or equal to 0.01 % and less than or equal to 0.1 % by weight.

6. The composition for use according to one of claims 1 to 5, **characterized in that** the myrtle extract is a non-polar fraction consisting of myrtucommulones and ursolic acid.

7. The composition for use according to claim 6, **characterized in that** said myrtucommulones comprise mainly myrtucommulones A, B', D, B, isosemimyrtucommulone and semimyrtucommulone, advantageously with a total myrtucommulone content of between 3 % and 10 % by weight, in relation to the total weight of myrtle extract.

8. The composition for use according to claim 6, **characterized in that** the ursolic acid content is between 10 % and 30 % by weight, in relation to the total weight of myrtle extract.

9. The composition for use according to claim 6, **characterized in that** the ursolic acid content is less than 15 % by weight, in relation to the total weight of myrtle extract.

10. The composition for use according to one of claims 1 to 9, **characterized in that** the antibiotic is administered in topical form.

11. The composition for use according to one of claims 1 to 10, **characterized in that** the composition is used in the treatment of acne.

12. The composition for use according to one of claims 1 to 11, **characterized in that** the composition is used in the treatment of folliculitis decalvans.

13. A combination product consisting of a dermatological composition containing myrtle extract at a concentration of 0.01 % to 1 % by weight and a dermatological composition containing at least one antibiotic, for simultaneous, separate or sequential use in the treatment of acne or folliculitis decalvans.

14. The product according to claim 13, **characterized in that** the dermatological composition contains a quantity of myrtle extract greater than or equal to 0.01 % and less than or equal to 0.1 % by weight.
